# EUROPÄISCHE PATENTSCHRIFT

(11) **EP 1 152 763 B1**
(45) Veröffentlichungstag und Bekanntmachung des Hinweises auf die Patenterteilung: **04.12.2002**
(21) Anmeldenummer: 00910649.3
(22) Anmeldetag: 08.02.2000
(51) Int. Cl.: A61K 31/519, A61P 25/30

(54) **VERWENDUNG VON DESOXYPEGANIN ZUR BEHANDLUNG VON DROGENABHÄNGIGKEIT**
USE OF DESOXYPEGANINE FOR THE TREATMENT OF DRUG DEPENDENCE
UTILISATION DE LA DESOXYPEGANINE POUR LE TRAITEMENT DE LA TOXICOMANIE

(30) Priorität: 19.02.1999 DE 19906978
(43) Veröffentlichungstag der Anmeldung: 14.11.2001
(73) Patentinhaber: LTS Lohmann Therapie-Systeme AG, 56626 Andernach (DE); HF ARZNEIMITTELFORSCHUNG GmbH, 59368 Werne (DE)
(72) Erfinder: ASMUSSEN, Bodo, D-56170 Bendorf (DE); HILLE, Thomas, D-56567 Neuwied (DE); HOFFMANN, Hans-Rainer, D-56566 Neuwied (DE); OPITZ, Klaus, D-48157 Münster (DE)
(74) Vertreter: Schmidt, Werner, Dr.
(86) Internationale Anmeldenummer: EP0000974
(87) Internationale Veröffentlichungsnummer: WO00048582

(56) Entgegenhaltungen:
- WO-A-00/48579
- DE-A- 19 906 974
- DE-A- 19 906 975
- TULYAGANOV, N. ET AL: "The pharmacological characteristics of deoxypeganine hydrochloride" FARMAKOL. TOKSIKOL. (MOSCOW) (1986), 49(3), 37-40 , XP000957689
- MURATOVA, V. V. ET AL: "Toxicology of the new pharmaceutical preparation dehydroxypeganine hydrochloride" MED. ZH. UZB. (1984), (1), 53-5 , XP000957819
- DATABASE WPI Section Ch, Week 197913 Derwent Publications Ltd., London, GB; Class B02, AN 1979-25213B XP002151986 & SU 605 614 A (AS UZB CHEM GROWING), 6. April 1978 (1978-04-06) in der Anmeldung erwähnt

## Beschreibung

Desoxypeganin und / oder seine pharmazeutisch annehmbaren Säureadditionssalze können zur Behandlung von Drogenabhängigkeit verwendet werden. Diese Substanzen werden in einer kontrollierten Weise verabreicht, vorzugsweise in einer kontinuierlichen. Die pharmazeutische Darreichungsform ermöglicht eine kontrollierte Freisetzung für z. B. orale, transdermale oder auf anderem Wege parenterale Verabreichung.

Die Erfindung betrifft die Verwendung von Desoxypeganin sowie seiner pharmazeutisch geeigneten Säureadditionssalze zur Herstellung eines medikamentes zur Behandlung von Drogenabhängigkeit und / oder Drogensucht. Diese Verbindungen werden aus entsprechenden pharmazeutischen Formulierungen, die z. B. oral, transdermal oder anderweitig parenteral verabreicht werden, können z. B. kontinuierlich oder anderweitig kontrolliert freigesetzt. Solche Darreichungsformen können auch die subkutane, sublinguale oder intramuskuläre Verabreichung ermöglichen. Schließlich ist auch die Verabreichung als Implantat möglich. Der Begriff parenteral umfasst aber auch andere Anwendungsformen, ausser der oralen, also z. B. auch die rektale, intravenöse, intramuskuläre, intraperitoneale und nasale Anwendung.

Desoxypeganin oder ein pharmazeutisch annehmbares Säureaddiditonssalz oder eine Mischung aus Base und Salz dient zur Substitutionstherapie von Drogensüchtigen wie z. B. Opiatsüchtigen, beispielsweise Heroinsüchtigen oder Cocainsüchtigen.

Desoxypeganin ist in der ehemaligen Sowjetunion zwar ausführlich untersucht und seine pharmakologischen Wirkungen intensiv erforscht worden, die erfindungsgemässe Verwendung einer desoxypeganin-haltigen Formulierung zur Behandlung von Drogensucht und / oder Drogenabhängigkeit wurde aber bisher nicht beschrieben.

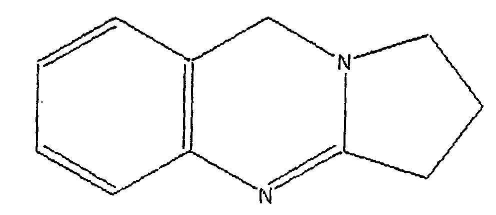

Bei Desoxypeganin (1,2,3,9-Tetrahydropyrrolo[2,1-b]chinazolin) handelt es sich um ein Alkaloid der Summenformel C₁₁H₁₂N₂ mit obiger Struktur, das in Pflanzen aus der Familie Zygophyllaceae enthalten ist. Als Säureadditionsssalz (Hydrochlorid) ist es kommerziell erhältlich.

Aufgrund seiner pharmakologischen Eigenschaften gehört Desoxypeganin zur Gruppe der reversibel wirkenden Cholinesterasehemmstoffe, steht in seinen Wirkungen dem Physostigmin und dem Neostigmin nahe, zeichnet sich jedoch durch besondere spezifische Eigenschaften aus. Desoxypeganin hemmt nämlich nicht nur die Acetylcholinesterase, sondern auch die Monoaminoxydase. Dieser Vorteil wiegt seine auf die Gewichtseinheit bezogene etwas geringere Cholinesterasehemmwirkung (im Vergleich zu Physostigmin) auf.

Im Gegensatz zu Neostigmin überwindet Desoxypeganin die Bluthirnschranke und antagonisiert die cerebralen Wirkungen cholinerger Gifte. Die Gewinnung des Desoxypeganin erfolgt durch Isolierung aus der Steppenraute (Peganum harmala) oder durch Synthese.

Arzneiformen, die Wirkstoffe kontrolliert freisetzen, sind im Stand der Technik bereits bekannt. Die Verabreichung pharmazeutisch wirksamer Verbindungen mittels solcher Formulierungen kann oral, transdermal oder anderweitig parenteral erfolgen. In derartigen Arzneimitteln kann das Desoxypeganin als solches oder in Form pharmazeutisch annehmbarer Säureadditonssalze vorliegen z. B. als Hydrohalogenid, insbesondere-chlorid oder -bromid, oder als Salz einer anderen pharmazeutisch annehmbaren Säure, z. B. als Citrat, Tartrat, Acetat. Diese Mittel enthalten ferner in der Regel Hilfsstoffe, wie Trägerstoffe, Fliessverbesserer, Lösungsmittel und Öle, deren Art und Menge je nach Darbietungsform schwankt. Im allgemeinen liegt der Gehalt an Wirkstoff im Arzneimittel, berechnet als freies Desoxypeganin, zwischen 0,1 und 50 Gew.-%, vorzugsweise zwischen 2 und 15 Gew.-%.

Der Wirkstoff bzw- das Wirkstoffgemisch wird durch die entsprechende pharmazeutische Formulierung über einen längeren Zeitraum abgegeben, z. B. für etwa 12, 16, 24, oder 72 Stunden. Bei speziellen Darreichungsformen kann sich der Wirkstoffabgabezeitraum auch über mehr als 3 Tage erstrecken.

Prinzipiell ist eine transdermale Gabe von Desoxypeganin und seinen pharmazeutisch annehmbaren Säureadditionssalzen einer oralen oder parenteralen Applikation vorzuziehen. Es ist klar, dass ein transdermales therapeutisches System (TTS) eine stark erhöhte Sicherheit in Bezug auf die missbräuchliche Anwendung bietet. So ist eine Extraktion des Wirkstoffs aus der TTS-Matrix ohne fachmännische Kenntnisse nicht möglich. Damit stellt eine missbräuchliche parenterale Applikation durch einen Süchtigen zur Suchtbefriedigung eine wesentlich geringere Gefahr dar, als sie beispielsweise bei einer oral anzuwendenden Lösung gegeben wäre. Eine Therapie mit Hilfe eines transdermalen therapeutischen Systems kann ohne direkte Aufsicht bzw. ohne den Arzt durchgeführt werden. Ein weiterer Vorteil ist die direkte Kontrolle der abgegebenen Dosis über die Freisetzungsfläche. Bei einer Entzugstherapie lassen sich die notwendigen Dosen in einfacher Weise auf die jeweiligen Bedürfnisse des Süchtigen abstimmen. Daneben sind die bekannten Vorteile einer transdermalen Applikation gegeben, und zwar:
- Vermeidung der bei oraler Anwendung erforderlichen hohen Arzneistoffdosis, die dem First-Pass-Effekt Rechnung zu tragen hat, sowie
- besser steuerbare Blutspiegel.

Ein transdermales therapeutisches System (TTS) für die Verabreichung von Desoxypeganin und / oder eines seiner pharmazeutisch annehmbaren Säureadditionssalze als Wirkstoff zur Behandlung von Drogenabhängigkeit oder Drogensuchtham
- eine selbstklebende schichtförmige Matrix mit einem Gehalt an Wirkstoff(en) enthalten, wobei an oder auf der einen Seite der Matrix
- eine Abdeckfolie (backing liner) und an oder auf der anderen Seite der Matrix
- eine Abziehfolie (release liner) vorgesehen ist.

Gegebenenfalls kann das transdermale therapeutische System (TTS) für die Verabreichung von Desoxypeganin und / oder eines seiner pharmazeutisch verträglichen Salze als Wirkstoff zur Behandlung von Drogenabhängigkeit und / oder Drogensucht mit einer äusseren Abdeckschicht, einem Reservoir für den Wirkstoff, einem Klebeelement für den Hautkontakt des Pflasters und einer entfernbaren Schutzschicht versehen werden, wobei das Reservoir gegebenenfalls neben dem Wirkstoff Permeationsförderer, Stabilisatoren, Emulgatoren, Verdickungsmittel und / oder andere übliche Hilfsstoffe enthält.

Das transdermale therapeutische System kann durch einen Gehalt von 0,1 bis 50 Gew.-% Desoxypeganin, vorzugsweise 2 bis 20 Gew.-% Desoxypeganin auf Basis der Matrix oder des Reservoirs des applikationsfähigen Pflasters gekennzeichnet sein. Besonders bevorzugt ist ein Gehalt an 5 bis 20 Gew.-% Desoxypeganin.

Die Abdeckfolie des transdermalen therapeutischen Systems kann aus Polyester, Polypropylen, Polyethylen oder Polyurethan, jeweils gegebenenfalls metallisiert, und die Abziehfolie aus Polyester, Polypropylen oder beschichtetem Papier bestehen.

Für die Matrix eines transdermalen therapeutischen Systems kommen Druckhaftkleber oder Haftschmelzkleber auf Basis von Polyacrylat, Polyisobutylen, Silikon, Styrol/Butadien Copolymerisat oder Styrol/Isopren-Copolymerisat in Frage.

Das transdermale therapeutische System kann durch eine semipermeable Membran gekennzeichnet sein, insbesondere eine die Geschwindigkeit des Wirkstofffreisetzung steuernde Membran. Die Membran kann auf Basis von Silikon, Polypropylen, Ethylen-Vinylacetat oder Polyvinylacetat vorgesehen werden.

Bei dem transdermalen therapeutischen System kann das Klebeelement in Form einer das Reservoir (sofern keine Membran vorgesehen ist) oder in Form einer die Membran vollständig oder nur an ihrer Peripherie ringförmig abdeckenden Schicht vorgesehen sein. Für das Klebelement kann ein druckempfindliches Klebemittel auf Silikon- oder Acrylatbasis verwendet werden.

Durch die Verabreichung von Desoxypeganin sowie seinen pharmazeutisch geeigneten Säureadditionssalzen oder einem Gemisch von Base und Salz werden die Symptome der Drogenabhängigkeit und / oder Drogensucht, insbesondere die physischen, aber auch die psychischen Entzugserscheinungen verringert bzw. ausgeschaltet.

Die Erfindung betrifft die Verwendung von Desoxypeganin und / oder eines pharmazeutisch annehmbaren Salzes von Desoxypeganin zur Herstellung einer Darreichungsform (Arzneimittels) mit verzögerter Freisetzung dieses Wirkstoffs oder Wirkstoffgemischs für die Behandlung der psychischen und /oder physischen Entzugssymptome von Drogensucht und / oder Drogenabhängigkeit.

## Patentansprüche

1. Verwendung von Desoxypeganin und / oder eines seiner pharmazeutisch verträglichen Säureadditionssalze zur Herstellung eines Arzneimittels zur Behandlung von Drogensucht und / oder Drogenabhängigkeit.

2. Verfahren zur Herstellung einer pharmazeutischen Darreichungsform zur Behandlung von Drogensucht und / oder Drogenabhängigkeit, **dadurch gekennzeichnet, dass** eine wirksame Menge Desoxypeganin und / oder eines seiner pharmazeutisch verträglichen Säureadditionssalze in fester Form oder in Lösung oder als Dispersion in einem anorganischen oder organischen Medium in die Formulierung eingebracht wird, wobei übliche Zusatzstoffe zugesetzt werden können.

## Claims

1. The use of deoxypeganine and/or one of its pharmaceutically tolerable acid addition salts for producing a medicament for the treatment of drug addiction and/or drug dependence.

2. A process for producing a pharmaceutical administration form for the treatment of drug addiction and/or drug dependence, which comprises incorporating into the formulation an efficacious amount of deoxypeganine and/or one of its pharmaceutically tolerable acid addition salts in solid form or in solution or as a dispersion in an inorganic or organic medium, it being possible to add customary additives.

## Revendications

1. Utilisation de la désoxypéganine et/ou de l'un de ses sels d'addition à un acide pharmaceutiquement acceptables pour la préparation d'un médicament destiné au traitement de la toxicomanie et/ou de la dépendance aux drogues.

2. Procédé de préparation d'une forme de présentation pharmaceutique destinée au traitement de la toxicomanie et/ou de la dépendance aux drogues, **caractérisé en ce qu'**une quantité efficace de désoxypéganine et/ou de l'un de ses sels d'addition à un acide pharmaceutiquement acceptables est introduite dans la formulation sous forme solide ou en solution ou en tant que dispersion dans un milieu inorganique ou organique, des additifs habituels pouvant être ajoutés.
